# EUROPEAN PATENT SPECIFICATION

(11) **EP 2 932 944 B1**
(45) Date of publication and mention of the grant of the patent: **04.12.2019**
(21) Application number: 15162915.1
(22) Date of filing: 09.04.2015
(51) Int. Cl.: A61F 5/01, A61N 1/36

(54) **SUPPORTING ORTHOSIS**
STÜTZORTHESE
ORTHÈSE DE SUPPORT

(30) Priority: 10.04.2014 DK 201470203
(43) Date of publication of application: 21.10.2015
(73) Proprietor: Bandagist Jan Nielsen A/S, 1360 Copenhagen K (DK)
(72) Inventor: Nielsen, Jan, 1360 Copenhagen K (DK)
(74) Representative: Larsen & Birkeholm A/S

(56) References cited:
- GB-A- 121 322
- US-A- 5 772 619
- US-A- 5 830 166
- US-A- 6 102 881
- US-A1- 2005 070 834
- US-A1- 2009 198 166
- US-A1- 2009 227 928
- US-A1- 2013 053 742

## Description

The present invention relates to a support orthosis

Orthoses are used as support for patients with reduced or no muscle function in knee and ankle after neurological damage such as for example peripheral nerve damage or damage to the central nervous system and also sclerosis or paralysis in parts of the body. Reduced or lacking muscle function may also be caused by acquired or congenital misalignment of the motility system. The purpose of orthoses is thus to support the affected extremity as well as possible, to achieve a gait pattern as normal as possible, and to remedy the patient's lacking proprioceptive sense, and to be able to control the foot.

To remedy the reduced or lacking muscle function, many forms of support devices for knee, ankle and foot are known. The known support orthoses may be made from leather, metal or laminate. The orthoses may also be manufactured from a combination of one or more of said materials, and many orthoses are made from rigid or stiff materials.

The known orthoses only provide fairly passive support to the patient and do not contribute to improving the patient's gait control or gait characteristics; further, a controlled ankle movement is preferable, to create increased blood supply, and to control the movement of the leg to avoid possible overextension of the patient's knee.

US 3805773 describes an orthosis to re-train the walking function of a patient. This orthosis comprises a rigid foot base connected with metal braces which extend along the side of the leg of a patient and are attached by Velcro straps. Each metal brace is equipped with a joint.

WO 2010/070364 A, US 2004/0260220 A1 and US 2012/0143112 A1 describe similar orthoses to mitigate, among other things, Drop Foot.

Swedish patent N° 520639 C2 describes an orthosis with a shoe insert which via a spring-loaded joint is connected with a metal brace that is connected to the leg of the patient just above the ankle.

US 2009/0198166 A1 discloses an ankle foot orthotic device for assisting a user during gait. The device comprises a foot support having a shape and size configured to the plantar aspect of a user foot, the foot support having a forward portion and a rearward portion, and a heel plate coupled to an upper surface of the rearward portion. The device further comprises an upper rod having a longitudinal axis and a lower end connected to an offset and a lower rod having a longitudinal axis, a lower end connected to the heel plate, and an upward end connected to the offset.

Although the above-mentioned support orthoses work adequately, the patients may however happen to overextend the knee, which leads to increased wear on the knee joint. Thus, there is a need for a support orthosis which can aid the patient in gaining better control of the ankle and the movement in the lower legs o that overextension may be avoided.

The objective of the invention is thus to provide a support orthosis which gives the patient an improved gait execution and improved dorsiflexion when walking, while at the same time supporting the leg so that overextension of the knee may be avoided.

The invention thus relates to a support orthosis for mitigation of Drop Foot for a patient - as set out in claim 1.

Contrarily to known support orthoses, where the support is designed so that it supports the leg at the side or on the calf, the support orthosis according to the invention is designed so that it supports the front of the leg along the tibia. As the support device additionally cooperates with a flexible foot plate, it is achieved that the patient can obtain a gait execution where the overextension of the knee joint may essentially be avoided.

The foot plate being flexible means that it is capable of flexing elastically at least in the toe-part and the heel-part. The toe-part and heel-part denote the area of the foot plate where the patient's toes and heel will be located when the patient is wearing the orthosis. The foot plate is, in one embodiment, capable of flexing about 0.5 cm to about 3 cm relative to the toe-part and the heel-part. That is to say that the toe-part and the heel-part from a resting position (where the foot plate carries no load) can be flexed upwards so that the part is lifted about 0.5 cm to about 3 cm in relation to the resting position. The bend is elastic, which means that energy is stored in the foot plate, which is released when the toe-part or the heel-part flexes back to the resting position. With this, the flexible foot plate assists the patient's gait execution. The middle part of the foot plate, between the toe-part and the heel-part may be designed to be relatively stiff. The expression "relatively stiff" means that the middle part of the foot plate is either entirely inflexible or can flex only a few millimetres. This may be achieved for example by increasing the material thickness in the middle part of the foot plate compared to the material thickness in the toe-part and the heel-part. The foot plate may be designed so that the toe-part and the heel-part are capable of flexing upwards but are less likely to bend downwards.

This may be achieved by manufacturing the foot plate in a fibre-composite material and orienting the fibres so that the desired properties are achieved.

To achieve the best possible support for the patient's leg and ensure a good gait execution, the support device is designed so that it extends along most of the patient's tibia. The support orthosis shall be attachable to the leg in such a manner that it does not get displaced or slides on the patient's leg. In one embodiment, the support element is designed such that it can be positioned on the tibia and attached with straps to fit around the calf. Preferably, the orthosis is attached with two straps, one strap at the upper part below the knee, and one strap at the lower end above the ankle.

In addition to preventing knee collapse, i.e. overextension of the knee, it has also been observed that the orthosis which is placed at the front of the leg is easier to put on than traditional orthoses, especially for patients with paralyses, where the function of one arm is wholly or partly disabled. Also, friction and irritation to the leg of the patient are reduced, and the patient is able to rest more comfortably in the orthosis when standing.

The support orthosis also comprises a connection element with a joint that divides the connection element into two parts. One part of the connection element is connected at the one end to the support device and at the other end is connected to the joint. The other part is connected to the foot plate at the one end, and at the other end connected with the joint. The joint is preferably designed so that it allows movement in only one plane or dimension, that is that two parts of the connection element are pivotable in two directions (forth and back) around the joint.

Although the joint may be designed in many ways, the joint is preferably a hinge joint around which the two parts of the connection element are pivotable. Thus, the foot plate is also pivotable in relation to the support device. When a patient is wearing the support orthosis, this function makes it possible for the foot of the patient to move up and down in relation to the leg, thus allowing a more natural gait pattern.

The joint may comprise an energy-storing element that may be in the form of a spring, e.g. a blade spring, a spiral spring coil or a spring of elastic material, such as e.g. rubber or a polymer material. The energy-storing element can be built into the joint in such a manner that it will bring the joint to be in one of its outer positions when the joint is not under load.

The joint will normally not allow unlimited movement in two directions but will comprise two outer positions in between which the two parts of the connection element can pivot up to about 50° in relation to each other. Preferably up to 40°, and especially up to 35° in relation to each other.

The energy storing element preferably comprises a spring or is a polymer elastic unit comprising a controlling function, in that such units have excellent properties with regard to collecting, storing and releasing energy. The energy-storing element is preferably a spring and may for example be made of metal, or silicone, polymer, rubber or similar.

When the hinge in one embodiment of the support orthosis is equipped with an energy-storing element in the form of a spring, it will be preferable to position the spring in such a manner that it, when the support orthosis is not under load, brings the joint to an end position where the connection element which is connected to the foot plate, flexes in such a way that the foot plate is brought to flex upwards with the forefoot. When a patient is wearing the support orthosis, this function will help the patient lift his/her foot, and thereby promote an improved execution of the gait. Depending on how much help the patient needs, the force of the spring may be adjusted.

The support orthosis thus differs from the known support orthoses; in addition to the support device being placed at the front of the leg, it further comprises a hinge joint being integrated in the connection element, where the known orthoses typically have stiff elements connecting the foot support and the support device on the leg of the patient. The advantage of the new support orthosis is that the element connecting the foot plate and the support device may be adjusted specifically to move with the aid of the joint, and that the foot plate is flexible so that it helps the patient's gait execution and dorsiflexion and prevents the overextension of the knee. Thereby, the support orthosis contributes not only to support the patient's foot and ankle, but it also contributes to controlled execution of the gait and controlled dorsiflexion and prevents overextension.

The flexible foot plate differentiates the orthosis according to the invention from other known orthoses, where the foot plates are manufactured from rigid, inflexible materials. The foot plate is preferably designed as a plate that is under the patient's foot. The foot plate may be manufactured as a standard support plate, that afterwards may be individually adapted to the foot of the specific patient, so that it for example fits inside a shoe or similar. The flexible foot plate is pivotable as described above and has elastic properties that allow the foot plate to absorb energy, store it and release it again. Thereby, the foot plate can also contribute to improving the patient's gait execution.

The support device is designed to fit fairly tightly but at the same time flexibly around the patient's leg on the front of the lower leg. The support device may then be attached with straps that fit around the calf. The support device may be attached to the patient's leg with one or two U-shaped parts that fit around the patient's tibia and are attached with straps that fit around the patient's calf. When the support device includes two U-shaped parts, these are interconnected by a part extending along the front of the leg.

The support device shall be fairly stiff to be able to absorb the forces that are transferred during walking motion. A little flexibility may however be preferable to counteract any unwanted distortion of the knee. The possibility for sideways displacement of about 1 to about 3 cm between the lower and the upper part of the support device should give sufficient flexibility in the support device.

The support device may be manufactured from nylon material or similar. It is also possible to make the support device from a more rigid material, e.g. a composite material. This can be useful if the support device is designed to be manipulated using one hand, e.g. for patients that are wholly or partially paralysed in one side of the body because of brain damage. The support device can be tightened around the tibia and the calf using known means, such as a buckle, Velcro or similar. The U-shaped parts may be made so that they are sufficiently flexible to adapt to the patient's leg.

In a preferred embodiment of the support orthosis, the connection element extends between the foot plate and the support device in such a manner that the connection element mainly will run along the inner or outer sides of the patient's foot and leg. This way, it has been shown that the best control and comfort for the patient may be achieved, and the support orthosis may follow the patient's natural flexing of the foot around the ankle.

In order to function as intended with the new support orthosis, the connection element with the joint should be relatively stiff in the sideways direction. The possibility for sideways displacement of the support device with respect to the foot plate should be minimal and preferably should not be present. Thus, a relatively stable support orthosis is achieved, which provides good support and has good properties with respect to gait execution.

At the same time, with the relatively stable construction, it is achieved that the patient can use the freedom in the orthosis and transfer his/her weight much more easily during the gait cycle by learning to advance his/her pelvis in front of the body's centre of gravity and thus achieve an energy-saving gait cycle.

To additionally improve the patient's gait characteristics, the foot plate may comprise 1 to 4 levels. In most cases these levels may be built in as standard in the support plate, but in one embodiment it is possible to individually adapt the levels to each patient as execution levels according to the patient's gait characteristics. Generally, the foot plate should be flexible where the heel strikes, stiff in the middle and flexible in the toe area.

The foot plate may thus be manufactured from a relatively stiff inflexible material to improve the support to the patient's foot and prevent overextension. The toe part and the heel part, however, should be flexible as described above. The stiff inflexible material that may constitute the middle part of the foot plate may be a composite material, where the material thickness is increased so that the material becomes stiff and inflexible; the joint will then also contribute to prevent the overextension of the patient's leg, which could put a strain on the knee.

Preferably, the support plate is manufactured from a composite material, e.g. a composite material based on carbon fibre, glass fibre or polymer materials.

For additional improvement of the support to the patient, the support orthosis may one or more devices in the form of straps or bands for supporting the foot and/or ankle. A T-strap, for example, may be mounted to extend from the foot plate and around the foot of the patient to provide improved support. The connection element of the support orthosis may also be provided with a strap that may be attached around the ankle of the patient, thus supporting the ankle.

In one embodiment the support orthosis may comprise additional support devices, e.g. a knee or a thigh support. Here, the support orthosis according to the invention may also be adapted to patients that have a need for this additional support of the leg.

It is clear that various parts of the orthosis, such as the support plate and the cuff, may be equipped with padding made of foam material, leather or similar, to make the orthosis comfortable for the patient.

In connection with the invention, electric stimulation of muscle groups in leg and foot, for example FES (functional electronic stimulation), may also be used. The electronic stimulation may in practice be implemented by an electrode or a pressure sensor under the heel of the patient's foot, i.e. on heel part of the foot plate, and depending on the load applied to the heel, a signal may be transmitted to a device that can trigger an electrical stimulation of a given place on the patient's leg. Preferably, the electrical stimulation will be performed on the outer side of the patient's leg, with electrodes that are mounted in the U-shaped part which encases a part of the patient's leg. Transmission of electrical signals from the pressure sensor to the device may be done with wire(s) or wirelessly with the aid of a transmitter and a receiver.

With the invention, a support orthosis is provided which comprises a support device to be positioned at the front of the leg, said orthosis further comprising an element or controlling joint, which in cooperation with the support device and the foot plate can mitigate the problems that are connected to Drop Foot. In one embodiment, the orthosis can absorb the energy of the movement from the patient, storing the energy and then releasing at least a part of the energy to the patient, and thereby helping the patient to improved gait execution. As the foot plate in the support orthosis may also be designed with execution levels, i.e. with a flexible toe part and heel part, this way an improved support orthosis is provided, which may help the patient control rotational movement in foot and ankle, and achieve improved gait execution and dorsiflexion, and also counteract the overextension of the knee joint. As the support device is fitted at the front of the patient's leg, the patient also is able to rest more comfortably in the support orthosis when standing up.

In the following, the invention is described with reference to the drawing, where:
- Fig. 1: shows a support orthosis according to the invention;
- Fig. 2: shows the support orthosis from the side, where the connection element is attached to the foot plate;
- Fig. 3: shows the support orthosis seen from the other side;
- Fig. 4: shows an embodiment with an ankle strap; and
- Fig. 5: shows an embodiment with a T-strap.

In the figures, the same reference numbers refer to the same parts on the support orthosis.

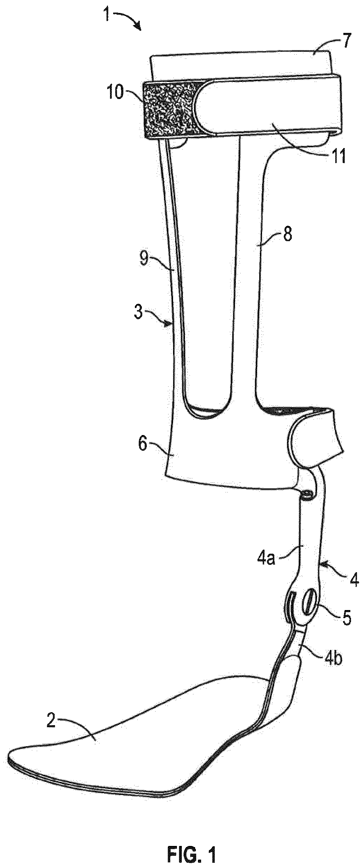

Figure 1 shows a support orthosis 1 according to the invention. The support orthosis 1 comprises several parts: a foot plate 2 to be placed under the patient's foot, a support device 3 to be fitted around the patient's leg, and also a connection element 4. The connection element 4 comprises a hinge joint 5, which divides the connection element into two parts, a first part 4a and a second part 4b.

The support device consists of two U-shaped parts 6 and 7, to be placed at the front side of the patient's leg (the tibia). In fig. 1, the cuffs or the U-shaped parts 6 and 7 are connected by two rod-shaped elements 8 and 9 which are shaped to follow a patient's leg. In alternative embodiments, the connection between the two U-shaped cuffs is a simple rod-shaped unit.

On part of their outer surface, the two U-shaped parts 6 and 7 are provided with velcro (velcro loops) strips 10 that can engage with velcro (velcro hooks) on straps 11 which fit around a patient's leg and thereby fasten the support device 3 to the front of the patient's leg. The support device 3 is thus intended to be positioned at the front of the patient's leg and thus enclose the tibia by the two U-shaped parts 6 and 7 to be attached by use of straps 11 that fit around the patient's calf. Other fastening means than Velcro may also be used, for example buckles or snap fasteners.

In figure 1, the support orthosis 1 is shown with the foot plate 2 in an approximately neutral position.

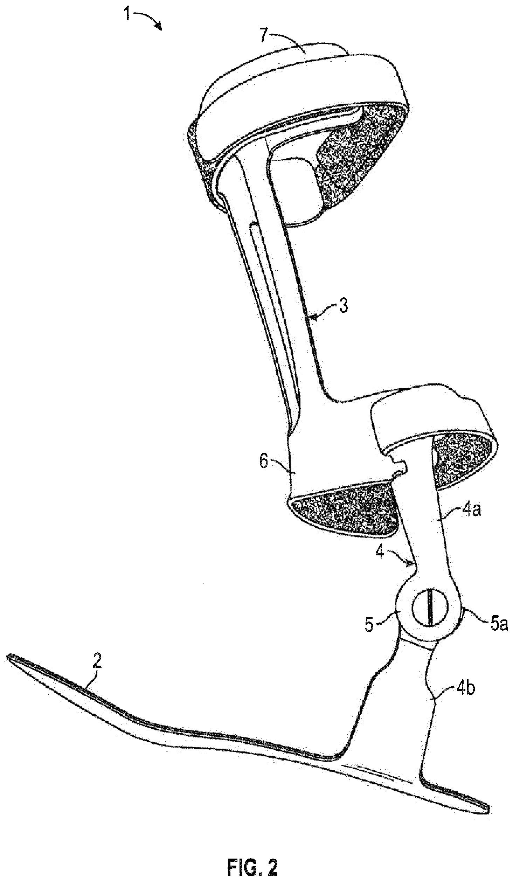

Figure 2 shows the same support orthosis 1, but here the foot plate 2 is flexed upwards, and the two parts 4a and 4b in the connection element 4 form an angle with each other since the connection element 4 is flexed around the joint 5. This is due to the fact that the joint 5 contains an energy-storing element, in this case an elastic element made from a polymer material, which is set to press the joint against one of its outer positions when there is no load on the joint. In the joint 5 the outer positions are defined by an edge 5a on the part of the joint connected to the part 4b, which may abut an edge in the part of the joint connected to the part 4a.

The part 4a is stiffly connected to the support device 3 via the U-shaped part 6. In the same way, the part 4b is stiffly connected to the foot plate 2. For this reason, the foot plate 2 will have an upward movement when the joint 5 is moved to one of its outer positions because of the influence from the energy-storing element. In the embodiment shown, a patient will therefore get additional help for execution of the gait because of the energy-storing element in the joint 5.

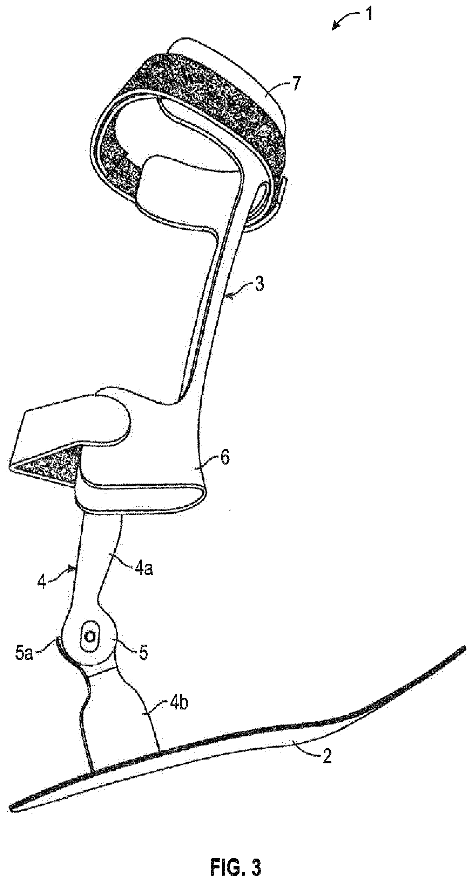

Figure 3 shows the support orthosis 1 from the opposite side compared to figure 2. Here the connection element 4 with the parts 4a and 4b is also shown. The joint 5 and one edge 5a, which serves to define the outermost positions of the joint, are also shown.

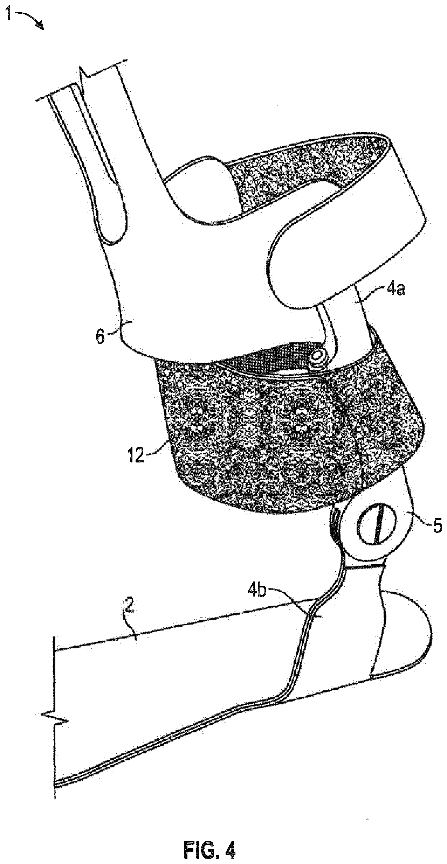

Figure 4 shows an embodiment where the support orthosis is equipped with an additional strap 12 for providing support the patient's ankle. The strap is attached to the upper part 4a of the connection element 4 and can be opened and closed with Velcro.

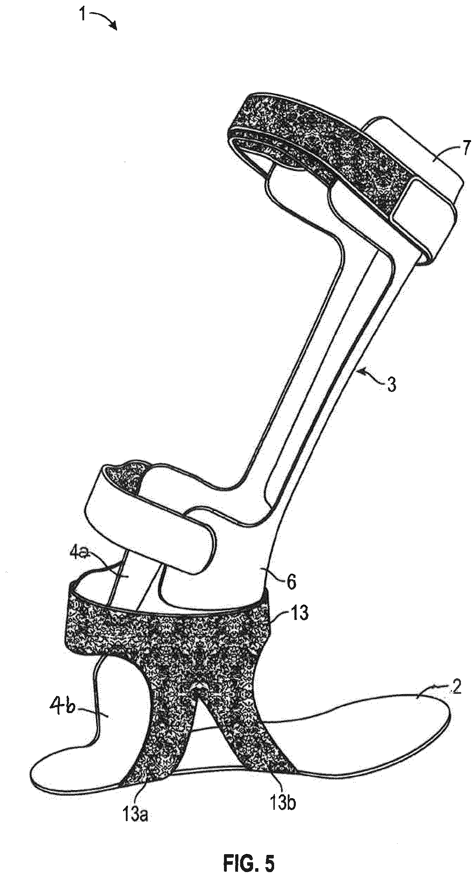

Figure 5 shows yet another embodiment of the support orthosis 1, where a so-called T-strap 13 is attached to the foot plate 2. The T-strap has, when open, approximately the form of a T. In the embodiment shown, the T-strap is attached in two places, 13a and 13b on the foot support 2, in that the lowermost part of the "T" has been separated in two.

The embodiments shown serve only to illustrate the principles in the invention. The foot plates according to the invention are centrally manufactured in e.g. three standard sizes that may later be adapted with flexibility in the toe-part and the heel-part. It is clear that each support orthosis shall be individually adapted to the specific patient, and that such adaptation is within the knowledge of the person skilled in the art. In the same way, the skilled person is able to adapt the energy-storing element, including elasticity, to the individual patient. The support orthosis has a size such that it can be worn by the patient inside a shoe, whereby the orthosis is comfortable and easy to use and also visually is less clearly visible than other known orthoses in the field.

## Claims

1. Support orthosis for mitigation of Drop Foot for a patient, comprising a foot plate (2) to be applied under a sole of a foot and a support device (3) to be attached on a lower leg of a patient, which support device (3) is designed to be mounted at the front of the patient's tibia, and which foot plate (2) is flexible, the foot plate (2) and the support device (3) are connected by at least one connection element (4) extending between the foot plate (2) and the support device (3) in such a manner that the connection element (4) runs along an inner or an outer side of a patient's foot and leg, which connection element (4) comprises a joint (5) dividing the connection element (4) into two parts, a first part (4a) and a second part (4b), which first part (4a) of the connection element (4) can be moved relative to the second part (4b) of the connection element (4) around the joint (5), **characterized in that** the support device (3) extends along most of the patient's tibia from just above the ankle to just below the knee and the joint (5) is a hinge joint allowing movement in only one plane, i.e. the first and second parts (4a, 4b) of the connection element (4) are pivotable in two directions (back and forth) around the joint.

2. Support orthosis according to claim 1, **wherein** the support device (3) comprises two U-shaped parts (6, 7) fitting around the patient's tibia and being attached with straps fitting around the patient's calf, the two U-shaped parts (6, 7) being interconnected by a part (8, 9) extending along the front of a patient's lower leg.

3. Support orthosis according to any of the previous claim, **wherein** the foot plate is flexible where the heel strikes, stiff in the middle and flexible in the toe area, e.g. the foot plate is capable of flexing elastically between 0.5 cm to 3 cm i.e. the toe-part and the heel-part may from a resting position where the foot plate carries no load be flexed upwards so that the toe- or heel-part is lifted between 0.5 cm to 3 cm relative to the resting position.

4. Support orthosis according to any of the previous claim, where the joint (5) comprises an energy-storing element, preferably chosen from a spring, e.g. a blade spring, a spiral spring or a spring made from an elastic material, such as e.g. rubber or a polymer material.

5. Support orthosis according claim 4, **wherein** the force of the energy-storing element can be adjusted.

6. Supporting orthosis according to any of the previous claims, where the foot plate (2) comprises 1 to 4 levels.

7. Support orthosis according to any of the previous claims, where the foot plate (2) is made from composite materials, preferably carbon fibre and resin.

8. Support orthosis according to any of the previous claims, where the joint has two outer positions, in between which outer positions the first and the second part (4a, 4b) of the connection element (4) can pivot up to about 50° relative to each other, preferably up to 40°, and e.g. up to 35° relative to each other.

9. Support orthosis according to claim 8, wherein the outer positions of the joint (5) are defined by a second edge (5a) of the part of the joint connected to the second part (4b) of the connection element (4) which second edge (5a) may abut a first edge of the first part (4a) of the joint (5).

10. Support orthosis according to any of the previous claims, which additionally comprises means for electrical stimulation of the muscles in the leg and/or foot of the patient.

## Patentansprüche

1. Stützorthese für die Linderung eines Spitzfußes für einen Patienten, die Folgendes umfasst: eine Fußplatte (2), die unter einer Sohle eines Fußes anzuwenden ist, und eine Stützvorrichtung (3), die an einen Unterschenkel eines Patienten anzubringen ist, wobei die Stützvorrichtung (3) so gestaltet ist, dass sie auf der Vorderseite des Schienbeins des Patienten befestigt wird, wobei die Fußplatte (2) flexibel ist, wobei die Fußplatte (2) und die Stützvorrichtung (3) durch mindestens ein Verbindungselement (4) verbunden sind, das sich zwischen der Fußplatte (2) und der Stützvorrichtung (3) auf eine Weise erstreckt, dass das Verbindungselement (4) entlang einer Innen- oder Außenseite eines Beins und Fußes eines Patienten verläuft, wobei das Verbindungselement (4) ein Gelenk (5), das das Verbindungselement (4) in zwei Teile teilt, einen ersten Teil (4a) und einen zweiten Teil (4b) umfasst, wobei der erste Teil (4a) des Verbindungselements (4) relativ zum zweiten Teil (4b) des Verbindungselements (4) um das Gelenk (5) herum bewegt werden kann, **dadurch gekennzeichnet, dass** die Stützvorrichtung (3) sich entlang des größten Teils des Schienbeins des Patienten von kurz über dem Knöchel bis kurz unter dem Knie erstreckt und das Gelenk (5) ein Scharniergelenk ist, das Bewegungen in nur einer Ebene ermöglicht, d. h. der erste und der zweite Teil (4a, 4b) des Verbindungselements (4) sind in zwei Richtungen (vorne und zurück) um das Gelenk herum drehbar.

2. Stützorthese nach Anspruch 1, **wobei** die Stützvorrichtung (3) zwei U-förmige Teile (6, 7) umfasst, die um das Schienbein des Patienten herum passen und mit Gurten um die Wade des Patienten herum befestigt werden, wobei die zwei U-förmigen Teile (6, 7) durch einen Teil (8, 9) miteinander verbunden werden, der sich entlang der Vorderseite des Unterschenkels eines Patienten erstreckt.

3. Stützorthese nach einem der vorhergehenden Ansprüche, **wobei** die Fußplatte flexibel ist, wo die Ferse auftritt, in der Mitte unbeweglich ist und im Zehenbereich flexibel ist, d. h. die Fußplatte ist in der Lage, sich zwischen 0,5 cm und 3 cm elastisch zu biegen, d. h. der Zehenteil und der Fersenteil können aus einer Ruheposition, in der die Fußplatte keine Belastung trägt, nach oben gebogen werden, sodass der Zehen- oder Fersenteil zwischen 0,5 cm und 3 cm relativ zur Ruheposition angehoben wird.

4. Stützorthese nach einem der vorhergehenden Ansprüche, wobei das Gelenk (5) ein Energiespeicherungselement umfasst, das vorzugsweise aus einer Feder gewählt ist, z. B. einer Blattfeder, einer Sprungfeder oder einer Feder, die aus einem elastischen Material gefertigt ist, wie z. B. aus einem Gummi- oder Polymermaterial.

5. Stützorthese nach Anspruch 4, **wobei** die Kraft des Energiespeicherungselements angepasst werden kann.

6. Stützorthese nach einem der vorhergehenden Ansprüche, wobei die Fußplatte (2) 1 bis 4 Ebenen umfasst.

7. Stützorthese nach einem der vorhergehenden Ansprüche, wobei die Fußplatte (2) aus Verbundwerkstoffen gefertigt ist, vorzugsweise aus Kohlenstofffaser und Harz.

8. Stützorthese nach einem der vorhergehenden Ansprüche, wobei das Gelenk zwei Außenpositionen hat, wobei der erste und der zweite Teil (4a, 4b) des Verbindungselements (4) zwischen diesen Außenpositionen relativ zueinander um bis zu 50° drehbar sind, vorzugsweise um bis zu 40° und z. B. um bis zu 35° relativ zueinander.

9. Stützorthese nach Anspruch 8, wobei die Außenpositionen des Gelenks (5) durch eine zweite Kante (5a) des Teils des Gelenks, der mit dem zweiten Teil (4b) des Verbindungselements (4) verbunden ist, definiert sind, wobei die zweite Kante (5a) an einer ersten Kante des ersten Teils (4a) des Gelenks (5) anliegen kann.

10. Stützorthese nach einem der vorhergehenden Ansprüche, die zusätzlich Mittel zur elektrischen Stimulation von den Muskeln im Bein und/oder Fuß des Patienten umfasst.

## Revendications

1. Orthèse de support pour l'atténuation du pied tombant pour un patient, comprenant une plaque de pied (2) à appliquer sous une plante d'un pied et un dispositif de support (3) à fixer sur une jambe inférieure d'un patient, lequel dispositif de support (3) est conçu pour être monté à l'avant du tibia du patient, et laquelle plaque de pied (2) est souple, la plaque de pied (2) et le dispositif de support (3) sont reliés par au moins un élément de liaison (4) s'étendant entre la plaque de pied (2) et le dispositif de support (3) de sorte que l'élément de liaison (4) longe un côté intérieur ou extérieur du pied et de la jambe d'un patient, lequel élément de liaison (4) comprend une articulation (5) divisant l'élément de liaison (4) en deux parties, une première partie (4a) et une seconde partie (4b), laquelle première partie (4a) de l'élément de liaison (4) peut être déplacée par rapport à la seconde partie (4b) de l'élément de liaison (4) autour de l'articulation (5), **caractérisée en ce que** le dispositif de support (3) s'étend le long de la plus grande partie du tibia du patient d'un emplacement juste au-dessus de la cheville à un emplacement juste en dessous du genou et **en ce que** l'articulation (5) est une articulation à charnière permettant un mouvement dans un seul plan, c'est-à-dire que les première et seconde parties (4a, 4b) de l'élément de liaison (4) peuvent pivoter dans les deux sens (aller et retour) autour de l'articulation.

2. Orthèse de support selon la revendication 1, **dans laquelle** le dispositif de support (3) comprend deux parties en forme de U (6, 7) se réglant autour du tibia du patient et étant fixées à l'aide de sangles se réglant autour du mollet du patient, les deux parties en forme de U (6, 7) étant reliés entre elles par une partie (8, 9) s'étendant le long de l'avant de la jambe inférieure d'un patient.

3. Orthèse de support selon l'une quelconque des revendications précédentes, **dans laquelle** la plaque de pied est souple là où le talon frappe, raide au milieu et souple au niveau de la zone d'orteil, par exemple, la plaque de pied est capable de se fléchir de manière élastique entre 0,5 cm et 3 cm, c'est-à-dire que la partie d'orteil et la partie de talon peuvent être fléchies vers le haut à partir d'une position de repos où la plaque de pied ne supporte pas de charge, de sorte que la partie d'orteil ou de talon est soulevée d'une distance entre 0,5 cm et 3 cm par rapport à la position de repos.

4. Orthèse de support selon l'une quelconque des revendications précédentes, dans laquelle l'articulation (5) comprend un élément accumulateur d'énergie, choisi de préférence parmi un ressort, par exemple un ressort à lame, un ressort en spirale ou un ressort en matériau élastique, comme par exemple un matériau en caoutchouc ou un matériau polymère.

5. Orthèse de support selon la revendication 4, **dans laquelle** la force de l'élément accumulateur d'énergie peut être réglée.

6. Orthèse de support selon l'une quelconque des revendications précédentes, dans laquelle la plaque de pied (2) comprend de 1 à 4 niveaux.

7. Orthèse de support selon l'une quelconque des revendications précédentes, dans laquelle la plaque de pied (2) est constituée de matériaux composites, de préférence de fibre de carbone et de résine.

8. Orthèse de support selon l'une quelconque des revendications précédentes, dans laquelle l'articulation a deux positions extérieures entre lesquelles la première et la seconde partie (4a, 4b) de l'élément de liaison (4) peuvent pivoter jusqu'à environ 50° l'une par rapport à l'autre, de préférence jusqu'à 40° et, par exemple, jusqu'à 35° l'une par rapport à l'autre.

9. Orthèse de support selon la revendication 8, dans laquelle les positions extérieures de l'articulation (5) sont définies par un second bord (5a) de la partie de l'articulation reliée à la seconde partie (4b) de l'élément de liaison (4), lequel second bord (5a) peut venir en butée contre un premier bord de la première partie (4a) de l'articulation (5).

10. Orthèse de support selon l'une quelconque des revendications précédentes, qui comprend en outre un moyen de stimulation électrique des muscles dans la jambe et/ou le pied du patient.
